# EUROPÄISCHE PATENTSCHRIFT

(11) **EP 1 309 319 B1**
(45) Veröffentlichungstag und Bekanntmachung des Hinweises auf die Patenterteilung: **23.03.2005**
(21) Anmeldenummer: 01971864.2
(22) Anmeldetag: 03.08.2001
(51) Int. Cl.: A61K 31/00

(54) **PHARMAZEUTISCHE, RAMIPRIL ENTHALTENDE BRAUSEFORMULIERUNG**
PHARMACEUTICAL, EFFERVESCENT FORMULATION CONTAINING RAMIPRIL
FORMULATION PHARMACEUTIQUE EFFERVESCENTE CONTENANT DU RAMIPRIL

(30) Priorität: 05.08.2000 DE 10038364
(43) Veröffentlichungstag der Anmeldung: 14.05.2003
(73) Patentinhaber: HEXAL AG, 83607 Holzkirchen (DE)
(72) Erfinder: KLOKKERS, Karin, Hexal AG, 83607 Holzkirchen (DE); FREUDENSPRUNG, Brigitte, Hexal AG, 83607 Holzkirchen (DE); HÄGER, Alexandra, Hexal AG, 83607 Holzkirchen (DE)
(86) Internationale Anmeldenummer: PCT/EP2001/008999
(87) Internationale Veröffentlichungsnummer: WO 2002/011709

(56) Entgegenhaltungen:
- US-A- 5 527 540

## Beschreibung

Die Erfindung betrifft eine lagerstabile, oral zu verabreichende pharmazeutische Brauseformulierung mit dem ACE-Hemmer Ramipril und/ oder dessen pharmazeutisch unbedenklichen Salzen und/ oder dessen pharmazeutisch unbedenklichen Estern als aktive Bestandteile mit einem Gewichtsverhältnis von der oder den Säure(n) zu Carbonat/Bicarbonat von 0,6 bis 1,3 und einem Gewichtsverhältnis von Ramipril zu Brausegemisch von 0,004 bis 0,013.

EP 079022 B1 beschreibt Ramipril, (2S,3aS,6aS)-1-[(S)-2-[[(S)-1-(Ethoxycarbonyl)-3-Phenylpropyl]amino]propanyl]octahydrocyclopenta[b]pyrrole-2-carbonsäure, und Ramiprilat als pharmazeutischen Wirkstoff.
Ramipril ist ein potentieller ACE-Hemmer, der zur Behandlung von essentieller Hypertonie, gering bis mäßig ausgeprägter Herzinsuffizienz und nach akutem Myocardinfarkt verwendet wird. Auf dem Markt befinden sich ausschließlich oral zu verabreichende Tabletten und Filmtabletten mit einem Ramiprilgehalt von 1,25 mg, 2,5 mg und 5 mg.
Die Patienten-Compliance spielt heutzutage eine sehr wichtige Rolle. Der Nachteil der gängigen oralen Darreichungsformen besteht darin, daß ein Großteil der Patienten unfähig ist, diese zu schlucken bzw. eine große Abneigung gegen diese Art der Darreichungsform besteht.

Schäumende bzw. brausende Präparate sind ein nützlicher und bevorzugter Präparatetyp für die orale Verabreichung. Bevor das Arzneimittel von dem Patienten geschluckt wird, wird ein Brausepräparat in beispielsweise einem wäßrigen Medium, wie Trinkwasser, gelöst und/ oder dispergiert. Die Lösung und/ oder Dispersion erfolgt sehr schnell, wobei das Brausen eine angenehme Präsentation des Arzneimittels ermöglicht, insbesondere bei Patienten, die es nicht vorziehen, Tabletten einzunehmen oder die Schwierigkeiten haben sie zu schlucken.

Bei der Herstellung einer üblichen Brauseformulierung gemäß dem Stand der Technik ("Die Tablette", Dr. W. A. Ritschel, Editio Cantor KG/ Aulendorf) mit Ramipril als Wirkstoff treten Probleme bezüglich der Lagerstabilität auf. Der Wirkstoff unterliegt einer erheblichen Zersetzung. Die Gehalte an Ramipril-Diketopiperazin und/ oder Ramiprildisäure, typische Zersetzungsprodukte von Ramipril, überstiegen schon nach kurzer Lagerzeit die in den Monographien angegebenen Maximalgehalte um ein vielfaches.

Dieses Stabilitätsproblem der ACE-Hemmer wird schon in EP 317 878 B1 beschrieben. Als eine wesentliche Zersetzungsursache gilt der mit dem Herstellungsverfahren verbundene Streß, vor allem dann, wenn der ACE-Hemmer im Gemisch mit Hilfsstoffen vorliegt. Der mechanische Streß erfolgt bei der Herstellung durch den bei der Tablettierung üblichen Preßdruck. Ein weiterer Faktor ist die bei der Lagerung auftretende Luftfeuchtigkeit oder gegebenenfalls auch eine erhöhte Temperatur. Außerdem wird die Art der verwendeten Tablettierhilfsstoffe als wesentlich für die Stabilität der ACE-Hemmer bezeichnet. Als Lösung des Problems beschreibt das Patent die Möglichkeit die Wirkstoffpartikel mit einer Polymerschutzhülle zu überziehen und/ oder einen Puffer in das System als Hilfsstoff zuzugeben, so daß sich in Gegenwart von Feuchtigkeit ein Mikro-pH-Wert von schwach sauer bis schwach alkalisch einstellt. Die Polymerschutzhülle erhält man durch Besprühen der Wirkstoffpartikel mit der Polymerlösung, was einen zusätzlichen Verfahrensschritt darstellt.

US-A-5527540 beschreibt eine Zubereitung, dadurch gekennzeichnet, dass getrennt voneinander je eine Brausephase und eine Wirkstoffphase hergestellt und anschliessend gemischt, sowie gegebenenfalls zu Tabletten verpresst werden, wobei sowie die Brausephase durch Einbetten pulverisierter Alkali- und/oder Erdalkalicarbonate bzw. -bicarbonate, als auch die Wirkstoffphase durch Einbetten pulverisierter Wirkstoffteilchen wie Ramipril, jeweils in Granulatkörner aus einer essbaren, organischen Säure, gebildet werden.

Die Aufgabe der Erfindung ist es nun, eine lagerstabile, schnell in Wasser zu lösende pharmazeutische Brauseformulierung zur Anwendung von Ramipril und/ oder dessen pharmazeutisch unbedenklichen Salzen und/ oder dessen pharmazeutisch unbedenklichen Estern für alle betroffenen Personen bereitzustellen, um somit schneller den therapeutisch wirksamen Blutplasmaspiegel zu erreichen und die Patienten-Compliance durch leichtere Einnahme zu verbessern. Die Herstellung sollte einfach und mit den üblichen Hilfsstoffen ohne zusätzlichen Verfahrensschritte erfolgen.

Es wurde nun überraschend gefunden, daß eine lagerstabile pharmazeutische Brauseformulierung enthaltend Ramipril und/ oder dessen pharmazeutisch unbedenklichen Salze und/ oder dessen pharmazeutisch unbedenklichen Ester als aktiven Bestandteil erhalten werden kann, indem das Gewichtsverhältnis von der oder den Säure(n) zu Carbonat/Bicarbonat 0,6 bis 1,3, insbesondere 0,7 bis 1,1 beträgt. Besonders bevorzugt ist das Gewichtsverhältnis von Säure zu Carbonat/Bicarbonat von 0,7 bis 0,9. Das Verhältnis von

Ramipril bzw. entsprechende Mengen pharmazeutisch unbedenkliche Salze und/oder Ester zu Brausegemisch sollte zwischen 0,004 bis 0,013, insbesondere 0,006 bis 0,01 betragen. Unter Brausegemisch wird hier die Menge an Säure(n) plus Carbonat/Bicarbonat verstanden. Die möglichen Darreichungsformen enthalten die für pharmazeutische Brauseformulierungen üblichen Hilfsstoffe. Es werden keine Polymerschutzhüllen oder Puffer benötigt, um die Stabilität zu gewährleisten. Die Herstellung der Brauseformulierung kann somit nach üblichen Verfahren erfolgen.
In der erfindungsgemäßen Brauseformulierung kann als Brausegemisch das Carbonat und das Bicarbonat der Alkali- und/ oder der Erdalkalimetalle, z.B. Natriumcarbonat bzw. Natriumbicarbonat, Calciumcarbonat bzw. Calciumbicarbonat und/ oder Magnesiumcarbonat bzw. Magnesiumbicarbonat als Kohlendioxidquelle, in Verbindung mit mindestens einer Säure, z.B. Citronensäure, Mononatriumcitrat, Ascorbinsäure, Gluconsäure, Milchsäure, Äpfelsäure und Weinsäure, verwendet werden.

Das erfindungsgemäße Brausegemisch besteht aus 20-40 Gew.%, insbesondere 25-35 Gew.% mindestens einer physiologisch verträglichen Säure und 25-40 Gew.%, insbesondere 30-35 Gew.% mindestens eines physiologisch verträglichen Carbonats und 1-5 Gew.%, insbesondere 2-3 Gew.% mindestens eines physiologisch verträglichen Bicarbonats bezogen auf das Gesamtformulierungsgewicht.

In der bevorzugten erfindungsgemäßen Brauseformulierung wird Citronensäure mit einer Kombination von Natriumcarbonat und Natriumbicarbonat als Brausegemisch verwendet.

Das Gewichtsverhältnis des Brausegemisches von der oder den Säure(n) zu Carbonat/Bicarbonat kann dabei 0,6 bis 1,3, insbesondere 0,7 bis 1,1 betragen. Bevorzugt liegt das Verhältnis bei 0,7 bis 0,9, wobei das Verhältnis von Ramipril bzw. den entsprechenden Mengen dessen pharmazeutisch unbedenklichen Salzen und/oder Estern zu Brausegemisch zwischen 0,004 bis 0,013, insbesondere 0,006 bis 0,01 beträgt.

Die erfindungsgemäße Brauseformulierung kann Ramipril und/ oder dessen pharmazeutisch unbedenkliche Salze und/ oder dessen pharmazeutisch unbedenklichen Ester als aktive Bestandteile enthalten. Als pharmazeutisch unbedenkliche Salze von Ramipril werden Säureadditionssalze verstanden. Diese erhält man durch die Reaktion des freien Ramiprils mit pharmazeutisch unbedenklichen Säuren. Pharmazeutisch unbedenkliche Säuren sind anorganische Säuren (z.B. Salzsäure, Bromwasserstoffsäure, Schwefelsäure, Salpetersäure, Phosphorsäure) oder organische Säuren (z.B. Essig-, Propion-, Hydroxyessig-, Milch-, Brenztrauben-, Oxal-, Malein-, Malon-, Bernstein-, Fumar-, Äpfel-, Wein-, Citronen-, Methansulfon-, Ethansulfon-, Benzolsulfon-, p-Toluolsulfon-, Gyclohexansulfamin-, Salicyl-, p-Aminosalicyl- und Pamoasäure). Ebenso als Säureadditionssalze werden Solvate mit dem Wirkstoff bezeichnet. Derartige Solvate sind z.B. Hydrate, Alkoholate und dergleichen. Als weitere mögliche Salze von Ramipril kommen vor allem Alkalisalze, wie z.B. das Kalium-, Natrium- und Lithiumsalz, Erdalkalimetallsalze, wie z.B. Calcium- und Magnesiumsalz, sowie Zinksalze und Ammoniumsalze in Frage.

Die erfindungsgemäße Brauseformulierung kann pro Dosierungseinheit eine wirksame Menge an Ramipril von 1-15 mg, insbesondere von 1-10 mg enthalten.
Die bevorzugten erfindungsgemäße Brauseformulierungen enthalten 1,25; 2,5; 5 oder 10 mg Ramipril pro Dosierungseinheit.

Die erfindungsgemäße Brauseformulierung kann pro Dosierungseinheit eine wirksame Menge an pharmazeutisch unbedenklichen Salzen und/ oder dessen pharmazeutisch unbedenklichen Estern von Ramipril enthalten, die einem Gehalt von Ramipril von 1-15 mg, insbesondere von 1-10 mg, bevorzugt von 1,25 mg; 2,5 mg; 5 mg und 10 mg entspricht.

Ein akzeptabler Geschmack der resultierenden Lösung des Wirkstoffes kann durch Zusatz geeigneter Geschmackskorrigenten, wie Aromastoffen, Zucker, Saccharose bzw. Zuckeraustaustauschstoffen erreicht werden. Zucker oder Zuckeraustauschstoffe können in einer Menge von bis zu etwa 50 Gew.% vorliegen. Zur Herstellung der erfindungsgemäßen Brauseformulierung geeignete Geschmackskorrigenten sind künstliche oder natürliche Süßstoffe (0,5 bis 5 Gew.%), vorzugsweise Saccharin-Natrium, Natrium-Cyclamat, Sorbit, Aspartam, Acesulfam, Acesulfam-Kalium oder Mannit, oder künstliche oder natürliche Aromastoffe, vorzugsweise Zitronen-, Orange, Grapefruit, Kirsch, Banane-, Pfefferminze-, Karamel-, Vanille-, Waldfrucht- und Himbeeraroma. Die Aromen werden vorzugsweise in einer Menge von 0,5 bis 3 Gew.% verwendet.

Weiterhin werden nach dem Stand der Technik bekannte, pharmazeutische, wasserlösliche Hilfsstoffe zur Herstellung der erfindungsgemäßen Brauseformulierung, z.B. Füll- und Bindemittel [Mannit, Lactose (Tablettose, Ludipress), Dextrose (Emdex)] sowie Schmiermittel (Polyethylenglykole, Compritol, L-Leucin, Magnesiumstearat, Stearinsäure) eingesetzt. Als weitere mögliche Tablettenhilfsstoffe können gegebenenfalls ein oder mehrere Polysaccharide zugegeben werden. Bevorzugte Polysaccharide sind Cyclodextrine. Mögliche Vertreter stellen α-, β-, γ- Cyclodextrin und/ oder deren pharmazeutisch unbedenklichen Derivate, insbesondere β-Cyclodextrin, dar.

Die erfindungsgemäße Brauseformulierung kann in Form von Tabletten oder Pulver oder Granulat, welches in Sachets abgefüllt werden kann, vorliegen. Die bevorzugte Brauseformulierung liegt in Form von Tabletten vor.
Das Brausetablettengewicht kann 1-4 Gramm betragen, wobei ein Gewicht von 1,5-2,5 Gramm bevorzugt wird. Der Brausetablettendurchmesser kann 16-25 mm betragen.

Nach Auflösung der Brauseformulierung erhält man über einen gewissen Zeitraum eine stabile Lösung.

Die Erfindung wird durch nachstehende Beispiele näher erläutert.

### Beispiel 1:

| | |
|---|---|
| Ramipril | 10 mg/ Tabl. |
| Citronensäure | 550 mg/ Tabl. |
| Natriumcarbonat | 600 mg/ Tabl. |
| Natriumhydogencarbonat (Bicarbonat) | 50 mg/ Tabl. |
| Trinatriumcitrat | 30 mg/ Tabl. |
| Lactose | 400 mg/ Tabl. |
| Saccharin-Natrium | 5 mg/ Tabl. |
| Natrium-Cyclamat | 50 mg/ Tabl. |
| PEG 6000 | 170 mg/Tabl. |
| Aroma (Zitrone) | 50 mg/ Tabl. |

Das Gesamtgewicht einer Tablette beträgt 1915 mg.
Ramipril und PEG 6000 werden gemahlen und gesiebt, die restlichen Bestandteile werden zugemischt. Das Gemisch wird zu Tabletten mit 20 mm Durchmesser und 1915 mg verpreßt.
Nach dem Auflösen resultiert eine klare Lösung. Das Gewichtsverhältnis von Citronensäure zu Carbonat/Bicarbonat entspricht ca. 0,85.

### Beispiel 2:

| | |
|---|---|
| Ramipril | 10 mg/ Tabl. |
| Citronensäure | 600 mg/ Tabl. |
| Natriumcarbonat | 600 mg/ Tabl. |
| Natriumhydogencarbonat (Bicarbonat) | 50 mg/ Tabl. |
| Trinatriumcitrat | 30 mg/ Tabl. |
| Lactose | 400 mg/ Tabl. |
| Saccharin-Natrium | 5 mg/ Tabl. |
| Natrium-Cyclamat | 50 mg/ Tabl. |
| PEG 6000 | 170 mg/Tabl. |
| Aroma (Himbeere) | 50 mg/ Tabl. |

Das Gesamtgewicht einer Tablette beträgt 1965 mg.
Ramipril und PEG 6000 werden gemahlen und gesiebt, die restlichen Bestandteile werden zugemischt. Das Gemisch wird zu Tabletten mit 20 mm Durchmesser und 1965 mg verpreßt. Nach dem Auflösen resultiert eine klare Lösung. Das Gewichtsverhältnis von Citronensäure zu Carbonat/Bicarbonat entspricht ca. 0,92.

### Beispiel 3:

| | |
|---|---|
| Ramipril | 10 mg/ Tabl. |
| Citronensäure | 650 mg/ Tabl. |
| Natriumcarbonat | 600 mg/ Tabl. |
| Natriumhydogencarbonat (Bicarbonat) | 50 mg/ Tabl. |
| Trinatriumcitrat | 30 mg/ Tabl. |
| Lactose | 400 mg/ Tabl. |
| Saccharin-Natrium | 5 mg/ Tabl. |
| Natrium-Cyclamat | 50 mg/ Tabl. |
| PEG 6000 | 170 mg/Tabl. |
| Aroma (Banane) | 50 mg/ Tabl. |

Das Gesamtgewicht einer Tablette beträgt 2015 mg.
Ramipril und PEG 6000 werden gemahlen und gesiebt, die restlichen Bestandteile werden zugemischt. Das Gemisch wird zu Tabletten mit 20 mm Durchmesser und 2015 mg verpreßt. Das Gewichtsverhältnis von Citronensäure zu Carbonat/Bicarbonat entspricht ca. 1,0.

### Beispiel 4:

| | |
|---|---|
| Ramipril | 10 mg/ Tabl. |
| Citronensäure | 700 mg/ Tabl. |
| Natriumcarbonat | 600 mg/ Tabl. |
| Natriumhydogencarbonat (Bicarbonat) | 50 mg/ Tabl. |
| Trinatriumcitrat | 30 mg/ Tabl. |
| Lactose | 400 mg/ Tabl. |
| Saccharin-Natrium | 5 mg/ Tabl. |
| Natrium-Cyclamat | 50 mg/ Tabl. |
| PEG 6000 | 170 mg/Tabl. |
| Aroma (Karamel) | 50 mg/ Tabl. |

Das Gesamtgewicht einer Tablette beträgt 2015 mg.

Ramipril und PEG 6000 werden gemahlen und gesiebt, die restlichen Bestandteile werden zugemischt. Das Gemisch wird zu Tabletten mit 20 mm Durchmesser und 2065 mg verpreßt. Nach dem Auflösen resultiert eine klare Lösung. Das Gewichtsverhältnis von Citronensäure zu Carbonat/Bicarbonat entspricht ca. 1,08.

### Beispiel 5:

| | |
|---|---|
| Ramipril | 10 mg/ Tabl. |
| Citronensäure | 550 mg/ Tabl. |
| Natriumcarbonat | 650 mg/ Tabl. |
| Natriumhydogencarbonat (Bicarbonat) | 50 mg/ Tabl. |
| Trinatriumcitrat | 30 mg/ Tabl. |
| Lactose | 400 mg/ Tabl. |
| Saccharin-Natrium | 5 mg/ Tabl. |
| Natrium-Cyclamat | 50 mg/ Tabl. |
| PEG 6000 | 170 mg/Tabl. |
| Aroma (Waldbeere) | 50 mg/ Tabl. |

Das Gesamtgewicht einer Tablette beträgt 1965 mg.
Ramipril und PEG 6000 werden gemahlen und gesiebt, die restlichen Bestandteile werden zugemischt. Das Gemisch wird zu Tabletten mit 16 mm Durchmesser und 1965 mg verpreßt. Das Gewichtsverhältnis von Citronensäure zu Carbonat/Bicarbonat entspricht ca. 0,79.

### Beispiel 6:

| | |
|---|---|
| Ramipril | 10 mg/ Tabl. |
| Citronensäure | 550 mg/ Tabl. |
| Natriumcarbonat | 700 mg/ Tabl. |
| Natriumhydogencarbonat (Bicarbonat) | 50 mg/ Tabl. |
| Trinatriumcitrat | 30 mg/ Tabl. |
| Lactose | 400 mg/ Tabl. |
| Saccharin-Natrium | 5 mg/ Tabl. |
| Natrium-Cyclamat | 50 mg/ Tabl. |
| PEG 6000 | 170 mg/Tabl. |
| Aroma (Zitrone) | 50 mg/ Tabl. |

Das Gesamtgewicht einer Tablette beträgt 2015 mg.
Ramipril und PEG 6000 werden gemahlen und gesiebt, die restlichen Bestandteile werden zugemischt. Das Gemisch wird zu Tabletten mit 20 mm Durchmesser und 2015 mg verpreßt. Das Gewichtsverhältnis von Citronensäure zu Carbonat/Bicarbonat entspricht ca. 0,73.

### Beispiel 7:

| | |
|---|---|
| Ramipril | 10 mg/ Tabl. |
| Citronensäure | 600 mg/ Tabl. |
| Natriumcarbonat | 650 mg/ Tabl. |
| Natriumhydogencarbonat (Bicarbonat) | 50 mg/ Tabl. |
| Trinatriumcitrat | 3 0 mg/ Tabl. |
| Lactose | 400 mg/ Tabl. |
| Saccharin-Natrium | 5 mg/ Tabl. |
| Natrium-Cyclamat | 50 mg/ Tabl. |
| PEG 6000 | 170 mg/Tabl. |
| Aroma (Erdbeere) | 50 mg/ Tabl. |

Das Gesamtgewicht einer Tablette beträgt 2015 mg.
Ramipril und PEG 6000 werden gemahlen und gesiebt, die restlichen Bestandteile werden zugemischt. Das Gemisch wird zu Tabletten mit 20 mm Durchmesser und 2015 mg verpreßt. Das Gewichtsverhältnis von Citronensäure zu Carbonat/Bicarbonat entspricht ca. 0,86.

### Beispiel 8:

| | |
|---|---|
| Ramipril | 10 mg/ Tabl. |
| Citronensäure | 600 mg/ Tabl. |
| Natriumcarbonat | 700 mg/ Tabl. |
| Natriumhydogencarbonat (Bicarbonat) | 50 mg/ Tabl. |
| Trinatriumcitrat | 30 mg/ Tabl. |
| Lactose | 400 mg/ Tabl. |
| Saccharin-Natrium | 5 mg/ Tabl. |
| Natrium-Cyclamat | 50 mg/ Tabl. |
| PEG 6000 | 170 mg/Tabl. |
| Aroma (Zitrone) | 50 mg/ Tabl. |

Das Gesamtgewicht einer Tablette beträgt 2065 mg.
Ramipril und PEG 6000 werden gemahlen und gesiebt, die restlichen Bestandteile werden zugemischt. Das Gemisch wird zu Tabletten mit 20 mm Durchmesser und 2065 mg verpreßt. Das Gewichtsverhältnis von Citronensäure zu Carbonat/Bicarbonat entspricht ca. 0,8.

### Beispiel 9:

| | |
|---|---|
| Ramipril | 10 mg/ Tabl. |
| β-Cyclodextrin | 400 mg /Tabl. |
| Citronensäure | 600 mg/ Tabl. |
| Natriumcarbonat | 700 mg/ Tabl. |
| Natriumhydogencarbonat (Bicarbonat) | 50 mg/ Tabl. |
| Trinatriumcitrat | 30 mg/ Tabl. |
| Saccharin-Natrium | 5 mg/ Tabl. |
| Natrium-Cyclamat | 50 mg/ Tabl. |
| PEG 6000 | 170 mg/Tabl. |
| Aroma (Zitrone) | 50 mg/ Tabl. |

Das Gesamtgewicht einer Tablette beträgt 2065 mg.

Ramipril und PEG 6000 werden gemahlen und gesiebt, die restlichen Bestandteile werden zugemischt. Das Gemisch wird zu Tabletten mit 20 mm Durchmesser und 2065 mg verpreßt. Nach dem Auflösen resultiert eine klare Lösung. Das Gewichtsverhältnis von Citronensäure zu Carbonat/Bicarbonat entspricht ca. 0,8.

### Beispiel 10:

| | |
|---|---|
| Ramipril | 5 mg/ Tabl. |
| β-Cyclodextrin | 200 mg /Tabl. |
| Citronensäure | 300 mg/ Tabl. |
| Natriumcarbonat | 350 mg/ Tabl. |
| Natriumhydogencarbonat (Bicarbonat) | 25 mg/ Tabl. |
| Trinatriumcitrat | 15 mg/ Tabl. |
| Saccharin-Natrium | 5 mg/ Tabl. |
| Natrium-Cyclamat | 25 mg/ Tabl. |
| PEG 6000 | 85 mg/Tabl. |
| Aroma (Zitrone) | 25 mg/ Tabl. |

Das Gesamtgewicht einer Tablette beträgt 1035 mg.
Ramipril und PEG 6000 werden gemahlen und gesiebt, die restlichen Bestandteile werden zugemischt. Das Gemisch wird zu Tabletten mit 18 mm Durchmesser und 1035 mg verpreßt. Nach dem Auflösen resultiert eine klare Lösung. Das Gewichtsverhältnis von Citronensäure zu Carbonat/Bicarbonat entspricht ca. 0,8.

## Patentansprüche

1. Lagerstabile pharmazeutische Brauseformulierung, **gekennzeichnet durch** Ramipril und/oder dessen pharmazeutisch unbedenklichen Salze und/ oder dessen pharmazeutisch unbedenklichen Ester als aktive Bestandteile mit einem Gewichtsverhältnis von der oder den Säure(n) zu Carbonat/ Bicarbonat von 0,6 bis 1,3 und einem Gewichtsverhältnis von Ramipril und/oder entsprechende Mengen dessen pharmazeutisch unbedenkliche Salze und/oder Ester zu Brausegemisch von 0,004 bis 0,013.

2. Lagerstabile pharmazeutische Brauseformulierung nach Anspruch 1, **gekennzeichnet durch** Ramipril und/ oder dessen pharmazeutisch unbedenklichen Salze und/ oder dessen pharmazeutisch unbedenklichen Ester als aktive Bestandteile mit einem Gewichtsverhältnis von der oder den Säure(n) zu Carbonat/ Bicarbonat von 0,7 bis 1,1, bevorzugt von 0,7 bis 0,9.

3. Lagerstabile pharmazeutische Brauseformulierung nach Anspruch 1 und 2, **gekennzeichnet durch** ein Gewichtsverhältnis von Ramipril und/ oder entsprechende Mengen dessen pharmazeutisch unbedenkliche Salze und/ oder dessen pharmazeutisch unbedenkliche Ester zu Brausegemisch von 0,006 bis 0,01

4. Lagerstabile pharmazeutische Brauseformulierung nach Anspruch 1, 2 oder 3, **gekennzeichnet durch** ein Brausegemisch aus 20-40 Gew.%, insbesondere 25-35 Gew.% mindestens einer physiologisch verträglichen Säure und 25-40 Gew.%, insbesondere 30-35 Gew.% mindestens eines physiologisch verträglichen Carbonats und 1-5 Gew.%, insbesondere 2-3 Gew.% mindestens eines physiologisch verträglichen Bicarbonats bezogen auf das Gesamtformulierungsgewicht.

5. Lagerstabile pharmazeutische Brauseformulierung nach einem der vorangegangenen Ansprüche **gekennzeichnet durch** Citronensäure in Kombination mit Natriumcarbonat und Natriumbicarbonat als Brausegemisch.

6. Lagerstabile pharmazeutische Brauseformulierung nach einem der vorangegangenen Ansprüche, **gekennzeichnet durch** einen Gehalt von Ramipril von 1-15 mg, insbesondere 1-10 mg, bevorzugt 1,25 mg, 2,5 mg, 5 mg oder 10 mg pro Dosierungseinheit.

7. Lagerstabile pharmazeutische Brauseformulierung nach einem der vorangegangenen Ansprüche, **gekennzeichnet durch** einen Gehalt an pharmazeutisch unbedenklichen Salzen und / oder Estern von Ramipril, der einem Gehalt von 1-15 mg Ramipril, insbesondere 1-10 mg Ramipril, bevorzugt 1,25 mg, 2,5 mg, 5 mg oder 10 mg Ramipril pro Dosierungseinheit entspricht.

8. Lagerstabile pharmazeutische Brauseformulierung nach einem der vorangegangenen Ansprüche, **gekennzeichnet durch** einen fakultativen Gehalt an Süßstoff(en) und/oder Aromastoff(en).

9. Lagerstabile pharmazeutische Brauseformulierung nach einem der vorangegangenen Ansprüche, **gekennzeichnet durch** einen fakultativen Gehalt an Polysacchariden, insbesondere Cyclodextrin.

10. Lagerstabile pharmazeutische Brauseformulierung nach einem der vorangegangenen Ansprüche in Form von Tabletten, Pulver oder Granulat, welches in Sachets abgefüllt werden kann, insbesondere in Form von Tabletten.

11. Lagerstabile pharmazeutische Brauseformulierung nach Anspruch 1-10, **gekennzeichnet dadurch, daß** die Brauseformulierung als Lösung stabil ist.

## Claims

1. Storage-stable pharmaceutical effervescent formulation, **characterised by** ramipril and / or its pharmaceutically acceptable salts and / or its pharmaceutically acceptable esters as active components with a weight ratio of the acid(s) of 0.6 till 1.3 to carbonate / bicarbonate and a weight ratio of ramipril and / or corresponding quantities of its pharmaceutically acceptable salts and / or esters of 0.004 till 0.013 to the effervescent mixture .

2. Storage-stable pharmaceutical effervescent formulation according to claim 1, **characterised by** ramipril and / or its pharmaceutically acceptable salts and / or its pharmaceutically acceptable esters as active components with a weight ratio of the acid(s) of 0.7 till 1.1, preferable of 0.7 till 0.9, to carbonate / bicarbonate.

3. Storage-stable pharmaceutical effervescent formulation according to claims 1 and 2, **characterised by** a weight ratio of ramipril and / or corresponding quantities of its pharmaceutically acceptable salts and / or its pharmaceutically acceptable esters of 0.006 till 0.01 to the effervescent mixture.

4. Storage-stable pharmaceutical effervescent formulation according to claims 1, 2 or 3, **characterised by** an effervescent mixture of 20-40 wt%, particularly 25-35 wt% of at least one physiologically compatible acid and 25-40 wt%, particularly 30-35 wt% of at least one physiologically compatible carbonate and 1-5 wt%, particularly 2-3 wt% of at least one physiologically compatible bicarbonate, in relation to the total formulation weight.

5. Storage-stable pharmaceutical effervescent formulation according to one of the preceding claims, **characterised by** citric acid in combination with sodium carbonate and sodium bicarbonate as effervescent mixture.

6. Storage-stable pharmaceutical effervescent formulation according to one of the preceding claims, **characterised by** a content of ramipril of 1-15mg, particularly 1-10mg, preferably 1.25mg, 2.5mg, 5mg or 10mg per dosage unit.

7. Storage-stable pharmaceutical effervescent formulation according to one of the preceding claims, **characterised by** a content of pharmaceutically acceptable salts and /or esters of ramipril, which corresponds to a content of 1-15mg ramipril, particularly 1-10mg ramipril, preferably 1.25mg, 2.5mg, 5mg or 10mg ramipril per dosage unit.

8. Storage-stable pharmaceutical effervescent formulation according to one of the preceding claims, **characterised by** an optional content of sweetener(s) and / or flavouring(s).

9. Storage-stable pharmaceutical effervescent formulation according to one of the preceding claims, **characterised by** an optional content of polysaccharides, particularly cyclodextrin.

10. Storage-stable pharmaceutical effervescent formulation according to one of the preceding claims in form of tablets, powder or pellets, which can be filled in sachets, particularly in form of tablets.

11. Storage-stable pharmaceutical effervescent formulation according to claims 1-10, **characterised by** the fact that the effervescent formulation is stable as a solution.

## Revendications

1. Formule pharmaceutique effervescente stable au stockage, **caractérisée par** Ramipril et/ou ses sels ne présentant pas de risque pour la santé du point de vue pharmaceutique et/ou ses esters ne présentant pas de risque pour la santé du point de vue pharmaceutique comme principe actif avec un rapport pondéral du ou des acides par rapport au carbonate/bicarbonate de 0,6 à 1,3 et un rapport pondéral de Ramipril et/ou la quantité correspondante en sels et/ou en esters ne présentant pas de risque pour la santé du point de vue pharmaceutique par rapport au mélange effervescent de 0,004 à 0,013.

2. Formule pharmaceutique effervescente stable au stockage selon la revendication 1, **caractérisée par** Ramipril et/ou ses sels ne présentant pas de risque pour la santé du point de vue pharmaceutique et/ou ses esters ne présentant pas de risque pour la santé du point de vue pharmaceutique comme principe actif avec un rapport pondéral du ou des acides par rapport au carbonate/bicarbonate de 0,7 à 1,1, de préférence de 0,7 à 0,9.

3. Formule pharmaceutique effervescente stable au stockage selon les revendications 1 et 2, **caractérisée par** un rapport pondéral de Ramipril et/ou la quantité correspondante en sels ne présentant pas de risque pour la santé du point de vue pharmaceutique et/ou en esters ne présentant pas de risque pour la santé du point de vue pharmaceutique par rapport au mélange effervescent de 0,006 à 0,01.

4. Formule pharmaceutique effervescente stable au stockage selon les revendications 1, 2 ou 3, **caractérisée par** un mélange effervescent de 20 à 40 % en poids, en particulier par au moins 25-35 % poids d'un acide physiologiquement compatible, par au moins 30-35 % poids d'un carbonate physiologiquement compatible et par au moins 1-5 % poids, de préférence 2-3 % poids au moins d'un bicarbonate physiologiquement compatible par rapport au poids de la formule totale.

5. Formule pharmaceutique effervescente stable au stockage selon l'une des revendications précédentes, **caractérisée par** de l'acide citrique en combinaison avec du carbonate de sodium et du bicarbonate de sodium comme mélange effervescent.

6. Formule pharmaceutique effervescente stable au stockage selon l'une des revendications précédentes, c aractérisée par une teneur en Ramipril de 1-15 m g, en particulier 1-10 g, de préférence 1,25 mg, 2,5 mg, 5 mg ou 10 mg par unité de dosage.

7. Formule pharmaceutique effervescente stable au stockage selon l'une des revendications précédentes, **caractérisée par** une teneur en sels et/ou esters de Ramipril ne présentant pas de risque pour la santé du point de vue pharmaceutique qui correspond à une teneur de 1-15 mg de Ramipril, en particulier à 1-10 g de Ramipril, de préférence 1,25 mg, 2,5 mg, 5 mg ou 10 mg par unité de dosage.

8. Formule pharmaceutique effervescente stable au stockage selon l'une des revendications précédentes, **caractérisée par** une teneur facultative en édulcorant(s) et/ou aromatisant(s).

9. Formule pharmaceutique effervescente stable au stockage selon l'une des revendications précédentes, **caractérisée par** une teneur facultative en polysaccharides, en particulier en cyclodextrine.

10. Formule pharmaceutique effervescente stable au stockage selon l'une des revendications précédentes sous forme de comprimés, de poudre ou de granulés conditionnés en sachets, en particulier sous forme de comprimés.

11. Formule pharmaceutique effervescente stable au stockage selon les revendications 1-10, **caractérisée en ce que** la formule effervescente en tant que solution est stable.
